**Europäisches Patentamt**

**European Patent Office**

**Office européen des brevets**

(19)

(11) Veröffentlichungsnummer : **0 306 438 B1**

(12) **EUROPÄISCHE PATENTSCHRIFT**

(45) Veröffentlichungstag der Patentschrift :
08.04.92 Patentblatt 92/15

(51) Int. Cl.⁵ : **G01N 33/52, G01N 33/53**

(21) Anmeldenummer : **88730184.4**

(22) Anmeldetag : **15.08.88**

(54) **Vorrichtung zur Aufnahme einer Mehrzahl von Trägerelementen.**

(30) Priorität : **01.09.87 DE 8711962 U**

(43) Veröffentlichungstag der Anmeldung :
**08.03.89 Patentblatt 89/10**

(45) Bekanntmachung des Hinweises auf die
Patenterteilung :
**08.04.92 Patentblatt 92/15**

(84) Benannte Vertragsstaaten :
**AT BE CH DE ES FR GB GR IT LI LU NL SE**

(56) Entgegenhaltungen :
**EP-A- 0 087 899**
**US-A- 3 692 486**

(73) Patentinhaber : **Köhler, Dora**
**Kreutzer Weg 11**
**W-1000 Berlin 45 (DE)**
Patentinhaber : **Hermann, Brigitte, Dr.-med.**
**Bronnbachergasse 18a**
**W-8700 Würzburg (DE)**

(72) Erfinder : **Köhler, Dora**
**Kreutzer Weg 11**
**W-1000 Berlin 45 (DE)**
Erfinder : **Hermann, Brigitte, Dr.-med.**
**Bronnbachergasse 18a**
**W-8700 Würzburg (DE)**

(74) Vertreter : **Pfenning, Meinig & Partner**
**Kurfürstendamm 170**
**W-1000 Berlin 15 (DE)**

## Beschreibung

Die Erfindung bezieht sich auf eine Vorrichtung nach dem Oberbegriff des Anspruchs 1.

Bei einer Vielzahl von Untersuchungen und Testreihen im medizinischen Bereich, so beispielsweise in der Serologie, sind flüssige Proben verschiedenster Art unter vorgegebenen Bedingungen mit Testreagenzien zusammenzubringen, wobei nach vorgegebener Reaktionszeit durchgeführte Messungen zu auswertbaren Testergebnissen führen.

Für das angegebene Beispiel der Durchführung serologischer Untersuchungen sind in diesem Zusammenhang Mikrotiter bekannt, die aus einer Vielzahl von in einer Kunststoffkarte angeordneten, nebeneinanderliegenden Vertiefungen bestehen, in denen Serum mit Antigenen oder Antikörpern in Verbindung gebracht untersucht wird. Um mit Antigenen oder Antikörpern beschichtete Träger für solche Untersuchungen mit einem möglichst geringen technischen und materiallen Aufwand vereinfacht durchführen zu können, ist es bekannt, sich eines vorzugsweise aus Nitrozellulose oder einem entsprechenden Material bestehenden Trägers zu bedienen, der im wesentlichen aus einem streifenartigen Halteelement besteht, entlang dessen einer Längskante kammartig eine Vielzahl von mit den jeweiligen Antigenen oder dergleichen Testagenzien beaufschlagbare Streifen angeklebt werden können, deren Abstand voneinander dem Abstand der Vertiefungen innerhalb eines Gefäßrahmens mit matrixartig angeordneten Vertiefungen entspricht (DE-OS 34 16 933). Für die Versuchsdurchführung werden solche Träger auf den Gefäßrahmen aufgelegt, wobei die Teststreifen jeweils über eine Reihe der Vertiefungen in den Gefäßrahmen eingreifen, so daß für das gewählte Beispiel aus der Serologie mittels eines Trägers die Gesamtheit der an diesem befestigten Streifen und damit die daran befindlichen Antigene mit dem Serumproben innerhalb einer Vertiefungsreihe in Reaktion treten können.

Es hat sich jedoch gezeigt, daß insbesondere dann, wenn ein Vielzahl von zeilenweise nebeneinander bzw. hintereinander anzuordnenden Trägern auf den Gefäßrahmen aufgesetzt wird, diese dort nicht eindeutig genug und in der erforderlichen Weise unverwechselbar für die erforderliche Reaktionszeit arretiert werden können. Auch bleibt die Behälteranordnung bei dieser Art des Aufsetzens der Träger auf den Gefäßrahmen oben im wesentlichen offen, so daß sich beispielsweise hinsichtlich der für den Reaktionsablauf erforderlichen Feuchte nicht ausreichend konstante Bedingungen vorgeben lassen.

Weiterhin ist aus der EP-A-87 899 eine Vorrichtung bekannt, die aus einer Mikrotiterplatte mit einem Auflageflansch und einer auf diese aufsetzbaren, mit im aufgesetzten Zustand zur Mikrotiterplatte hin weisenden Stiften versehenen Abdeckung besteht. Die Anordnung der Stifte an der Abdeckung entspricht der Anordnung. der Vertiefungen in der Mikrotiterplatte. Die Stifte sind jeweils mit Köpfen ausgestattet, die geeignet sind, immunogenische Substanzen zu binden. Wenn die Abdeckung auf den Auflageflansch der Mikrotiterplatte aufgesetzt wird, greifen die Köpfe in deren flüssige Proben enthaltende Vertiefungen ein und man erhält auf diese Weise ein geschlossenes Testsystem. Diese Druckschrift betrifft jedoch das Testsystem selbst und nicht eine Vorrichtung zur Aufnahme und Handhabung eines Testsystems, bei dem nicht zwingend eine Mikrotiterplatte eingesetzt werden muß.

Hier setzt die vorliegende Erfindung ein, der die Aufgabe zugrunde liegt, eine Vorrichtung der eingangs genannten Art dahingehend weiterzuentwickeln und zu verbessern, daß die gewünschten Reaktionsbedingungen der jeweiligen Testphase bei eindeutig definierter Arretierung und Zuordnung der einzelnen Probenkomponenten zueinander innerhalb eines in sich geschlossenen Volumens vorgebbar sind.

Die Lösung dieser Aufgabe wird erfindungsgemäß durch die im Kennzeichen des Anspruchs 1 angegebenen Merkmale erreicht.

Vorteilhafte Ausgestaltungen und Weiterbildungen dieser Aufgabenlösung ergeben sich aus den Unteransprüchen.

Dadurch, daß der Wechseleinsatz in fixierter Lage in eine Feuchtewanne eingesetzt bzw. auf diese aufgesetzt und gemeinsam mit der Wanne von einer Abdeckung übergriffen wird, ist zumindest für die Zeit der Versuchsdauer sichergestellt, daß sich für die Vielzahl der Titerstreifen an den Trägerelementen, die mit den Untersuchungsmedien innerhalb der Testkompartimente in der Mikrotiterplatte in Reaktionseingriff gebracht sind, solche physikalischen Parameter wie Feuchtigkeit, Temperatur und dergleichen aber auch chemische Bedingungen nicht ändern. Der umlaufende Auflageflansch der Feuchtwanne arretiert den Wechseleinsatz und dient gleichermaßen der Aufnahme der Mikrotiterplatte oder dergleichen.

Der Wechseleinsatz kann in vorteilhafter Weise in paralleler Nebeneinanderordnung eine Mehrzahl von kammartigen Trägerelementen einfach dadurch aufnehemen, daß diese in hierfür vorgesehene Führungslamellen eingesteckt werden, wobei sie sich auf die gleiche einfache Weise nach Abschluß der Versuchszeit entweder einzeln oder zusammen mit dem Wechselrahmen aus der Vorrichtung entnehmen lassen. Die Feuchtwannesteht dann unmittelbar für die nächste Versuchsreihe, falls dieses gewünscht, zur Verfügung.

Das Einführen der Trägerelemente ind ie einzelnen Führungslamellen wird dadurch erleichtert, daß diese in ihrem oberen Einführungsbereich einen konisch verlaufenden Führungsabschnitt aufweisen, der in Einführungsrichtung nach unten hin in eine im wesentlichen parallele Schlitzführung übergeht, die dann ihrerseits die Lage jedes einzelnen Trägerelementes innerhalb des zugehörigen Einsatzschlitzes definiert. Es ist weiter vorteilhaft, daß die Trägerelemente innerhalb ihrer Einsatzschlitze in den Führungslamellen seitlich dadurch unverrutschbar gehalten sind, daß auf beiden Seiten entlang der stirnseitigen Endflächen der Führungslamellen Seitenwandungen vorgesehen sind, die in ihrer Höhe die Höhe der Führungslamellen ausreichend übergreifen.

Weitere Vorteile und Einzelheiten der Erfindung sollen nachfolgend anhand eines bevorzugten Ausführungsbeispieles näher beschrieben werden, welches sich besonders vorteilhaft im Zusammenhang mit der Immundiagnostik verwenden läßt. Es soll jedoch darauf hingewiesen werden, daß die vorliegende Vorrichtung selbstverständlich für eine Vielzahl verschiedenartigster Versuchsreihen und damit für einen großen Anwendungsbereich geeignet ist. Sie ist auch nicht auf den Bereich der Humanmedizin beschränkt, sondern läßt sich gleichermaßen für die Tierdiagnostik einsetzen wie auch ganz allgemein für Untersuchungen im biologischen Bereich.

Es zeigen:

Fig. 1 eine perspektivische Darstellung der im wesentlichen dreiteiligen Vorrichtungen, wobei diese Teile nicht zusammengefügt sind,

Fig. 2 eine stirnseitige Draufsicht auf den Wechseleinsatz, und

Fig. 3 eine perspektivische Darstellung eines in die Schlitzeinsätze der Führungslamellen einzusetzenden kammartigen Trägerelementes.

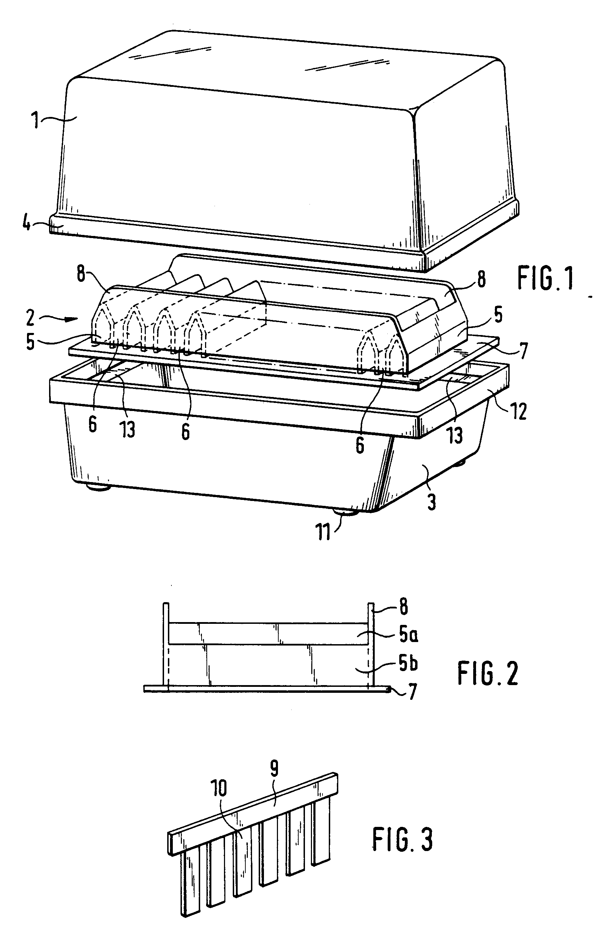

Der untere im Querschnitt rechtwinklige Behälter ist eine Feuchtwanne 3 mit im wesentlichen senkrechten Wänden, die in ihrem oberen Einsatzbereich durch einen Randflansch 12 gekennzeichnet ist, der eine umlaufende Auflageleiste 13 vorgibt. Die Auflageleiste 13 steht waagerecht aus der lichten Querschnittsfläche der Feuchtwanne 3 heraus und geht dann in einen senkrechten rahmenartigen oberen Verschlußrand über, wie das aus Fig. 1 ersichtlich ist.

Die so gebildete Auflageleiste 13 bzw. der Randflansch 12 dienen der Aufnahme und Arretierung des Wechseleinsatzes 2, dessen Schlitzplatte 7 sich von einem gewollten Spiel abgesehen dimensionsangepaßt in den Randflansch 12 einfügt. Der in bzw. auf die Feuchtwanne 3 ein-bzw. aufgesetzte Wechseleinsatz 2 besteht aus einer Mehrzahl von Führungslamellen 5, die sich in senkrechter Stellung von der Schlitzplatte nach oben weg erstrecken. Die Führungslamellen 5 übergreifen in ihrer Länge die Breite der Schlitzplatte 7, wobei sie in einem vorgegebenen Abstand vor den Randkanten dieser Platte enden, so daß sich auch hier ein umlaufender Rand für die Schlitzplatte 7 ergibt. Stirnseitig ist die Gesamtheit der Führungslamellen 5 auf jeder Seite von Seitenwandungen 8 übergriffen,die in ihrer Höhe über die Höhe der Führungslamellen hinausragen.

Der Querschnitt jeder Führungslamelle ist in seiner Form etwa mit der Giebelform eines Hauses vergleichbar und kennzeichnet sich durch einen oberen spitz zulaufenden konischen Führungsabschnitt 5a und einen sich nach unten bis zur Schlitzplatte 7 hin anschließenden Schlitzführungsabschnitt 5b. Mittels des oberen konischen Führungsabschnittes 5a wird das Einbringen der in Fig. 3 dargestellten kammartigen Trägerelemente in den Wechseleinsatz 2 erleichtert, während die sich daran anschließende Schlitzführung 5b jeweils einen Einsatzschlitz 6 zwischen den Führungslamellen definiert, der in seiner Breite hinlänglich auf die Dicke der kammartigen Trägerelemente 9 bzw. deren obere Halterung abgestimmt ist. Es ist in diesem Zusammenhang vorteilhaft, wenigstens die Bereiche der Schlitzführungen 5b mit zusätzlichen senkrecht verlaufenden Führungsnoppen zu versehen, mittels derer eine noch bessere Arretierung der Trägerelemente innerhalb der Einsatzschlitze 6 möglich ist. Am unteren Ende der Einsatzschlitze 6 im Bereich der Schlitzplatte 7 werden diese in Längsrichtung des Wechseleinsatzes verlaufend von im Abstand zueinander liegenden Stäben übergriffen, so daß nach Einführen der Trägerelemente in die Einsatzschlitze 6 deren Haltestreifen auf den Stegen aufliegen und damit die Trägerelemente auch in ihrer Eintauchtiefe definiert sind.

Die kammartigen Trägerelemente 9 mit ihren Halterungen und den Teststreifen 10 wie in Fig. 3 dargestellt sind nicht Gegenstand des vorliegenden Schutzbegehrens und werden daher hier nicht näher beschrieben. Entsprechendes gilt für die gleichfalls von dem Wechseleinsatz 2 aufgenommene Mikrotiterplatte.

Nach Einsetzen der mit den kammartigen Trägerelementen 9 bestückten Führungslamellen 5 und damit des Wechseleinsatzes 2 in die Feuchtwanne 3 wird diese von einer Abdeckung 1 verschlossen, wobei eine umlaufende Schließkante 4 der Abdeckung über den Randflansch 12 greift und sich auf diesen paßdicht aufsetzt. Am Boden der Feuchtwanne 3 finden sich vorteilhaft Fußansätze 11.

## Patentansprüche

1. Vorrichtung zur Aufnahme einer Mehrzahl von Trägerelementen, die ihrerseits kammartig eine Vielzahl von Titerstreifen oder dergleichen halten, die in entsprechend angeordnete, voneinander getrennte Testkompartimente innerhalb eines Gefäßrahmens, wie einer Mikrotiterplatte einführbar sind,
**gekennzeichnet durch**

a) eine Feuchtwanne (3), bestehend aus einem im Querschnitt rechteckigen Behälter mit einem im Verschlußbereich aus der Einsatzfläche des Behälters herausgeführten, umlaufenden Auflageflansch (12),

b) einem in die Feuchtwanne (3) in fixierter Lage einsetzbaren Wechseleinsatz (2) mit in Längsrichtung der Feuchtwanne (3) im Abstand zueinander angeordneten Führungslamellen (5) mit Einsatzschlitzen (6) für die Trägerelemente (9), und

c) durch eine die Feuchtwanne (3) und den Wechseleinsatz (2) verschließend übergreifende Abdeckung (1).

2. Vorrichtung nach Anspruch 1, gekennzeichnet durch eine Auflageleiste (13) im oberen Wandungsbereich der Feuchtwanne (3), die im Querschnitt waagerecht verlaufend aud dem Wannenrand herausführt.

3. Vorrichtung nach Anspruch 1, dadurch gekennzeichnet, daß der Wechseleinsatz (2) aus einer auf die Auflagesleiste (13) auflegbaren Schlitzplatte (7) besteht, auf der sich senkrecht nach oben gerichtete Führungslamellen (5) als integrale Bestandteile der Schlitzplatte befinden.

4. Vorrichtung nach Anspruch 1 und 3, dadurch gekennzeichnet, daß die Führungslamellen (5) im Querschnitt aus oberen spitz zulaufenden konischen Führungsabschnitten (5a) und sich nach unten daran anschließenden, untereinander etwa parallel zueinander verlaufenden Schlitzführungen (5b) zusammensetzen.

5. Vorrichtung nach wenigstens einem der Ansprüche. 1 bis 4, dadurch gekennzeichnet, daß die Stirnseiten der Führungslamellen (5) im Abstand vom Rand der Schitzplatte (7) von die Höhe der Lamellen übergreifenden Seitenwandungen (8) begrenzt sind.

6. Vorrichtung nach wenigstens einem der Ansprüche 1 bis 5, dadurch gekennzeichnet, daß die Schlitze der Schlitzplatte (7) im unteren Bereich Arretierungsstege aufweisen.

## Claims

1. Device for receiving a plurality of supporting elements which, in their turn, hold a plurality of titre strips or the like in the manner of a comb that can be introduced into appropriately arranged test compartments situated within the frame of a vessel, such as a microtitre plate, and separated from one another,
**characterised by**

a) a wetting trough (3) comprising a container of rectangular cross section with a circumferential supporting flange (12) guided out of the insertion surface of said container in the closure region,

b) a change insert (2) which can be inserted in said wetting trough (3) in fixed position and which has guiding laminae (5) spaced apart from one another in longitudinal direction of said wetting trough (3) and having insertion slits (6) for said supporting elements (9) and by

c) a cover (1) lapping over said wetting trough (3) and said change insert (2) in closing manner.

2. Device according to Claim 1, characterised by a seating rim (13) in the upper wall region of said wetting trough (3), said rim, as viewed in cross section, being horizontal and leading out of the edge of said trough.

3. Device according to Claim 1, characterised in that the change insert (2) comprises a slitted plate (7) that can be placed on said seating rim (13) and on which vertically upwardly directed guiding laminae (5) are located as integral component parts of said slitted plate.

4. Device according to Claims 1 and 3, characterised in that the guiding laminae (5), as viewed in cross section, are composed of tapering upper conical guiding portions (5a) and downwardly adjoining slit guides (5b) which are approximately parallel to one another.

5. Device according to at least one of Claims 1 to 4, characterised in that the end faces of said guiding laminae (5), at a distance from the edge of said slitted plate (7), are bounded by side walls (8) lapping over the height of said laminae.

6. Device according to at least one of Claims 1 to 5, characterised in that the slits of said slitted plate (7) have locking webs in-their lower region.

## Revendications

1. Dispositif pour la réception d'une pluralité des éléments de support qui, de leur part, tiennent une pluralité des bandes-titre ou des choses pareilles à la manière d'un peigne, qui peuvent être introduites dans des compartiments de têt situés à l'intérieur du bâti d'un récipient tel qu'une plaque-microtitre, arrangés de manière appropriée et séparés l'un de l'autre,
**caractérisé par**

a) une cuve (3) à humecter qui comprend un réservoir à coupe transversale rectangulaire avec une bride (12) de support circonférentielle qui est guidée hors de la surface d'insertion dudit réservoir dans la région de clôture,

b) une pièce (2) de rechange qui peut être introduite dans ladite cuve (3) à humecter en position fixée et qui présente des lamelles (5) de guidage écartées l'une de l'autre'dans la direction longitudinale de ladite cuve (3) à humecter et ayant des fentes (6) d'insertion pour lesdits éléments (9) de support et par

c) un couvercle (1) recouvrant ladite cuve (3) à humecter et ladite pièce (2) de rechange de manière clôturante.

2. Dispositif selon Revendication 1, caractérisé par un listel (13) d'appui dans la région-paroi supérieure de ladite cuve (3) à humecter, le listel, en coupe transversale, étant horizontal et menant hors du bord de ladite cuve.

3. Dispositif selon Revendication 1, caractérisé en ce que la pièce (2) de rechange comprend une plaque (7) aux fentes qui peut être placée sur le listel (13) d'appui et sur laquelle des lamelles (5) de guidage, dirigées verticalement vers le haut, sont situées comme éléments intégraux de ladite plaque aux fentes.

4. Dispositif selon Revendications 1 et 3, caractérisé en ce que les laqelles (5) de guidage, en coupe transversale, se sont composées des sections (5a) de guidage supérieures, coniques et rétrécissantes ainsi que des guides-fente (5b) qui sont contigus à celles-ci vers le bas et approximativement parallèle l'un à l'autre.

5. Dispositif selon au moins une des Revendications 1 à 4, caractérisé en ce que les côtés frontaux des lamelles (5) de guidage, à une distance du bord de ladite plaque (7) aux fentes, sont bordés par des parois (8) latérales recouvrant la hauteur des lamelles.

6. Dispositif selon au moins une des Revendications 1 à 5, caractérisé en ce que les fentes de la plaque (7) aux fentes présentent des barrettes d'arrêt dans leur région inférieure.

FIG.1

FIG.2

FIG.3